# EUROPÄISCHE PATENTSCHRIFT

(11) **EP 3 253 422 B1**
(45) Veröffentlichungstag und Bekanntmachung des Hinweises auf die Patenterteilung: **04.09.2019**
(21) Anmeldenummer: 15813435.3
(22) Anmeldetag: 18.12.2015
(51) Int. Cl.: A61L 2/18

(54) **REINIGUNGSVORRICHTUNG FÜR KLEINTEILE**
CLEANING DEVICE FOR SMALL PARTS
DISPOSITIF DE NETTOYAGE POUR PETITES PIÈCES

(30) Priorität: 18.12.2014 CH 19732014
(43) Veröffentlichungstag der Anmeldung: 13.12.2017
(73) Patentinhaber: Netzhammer, Eric, 4144 Arlesheim (CH)
(72) Erfinder: Netzhammer, Eric, 4144 Arlesheim (CH)
(74) Vertreter: Braun, André jr.
(86) Internationale Anmeldenummer: PCT/EP2015/080440
(87) Internationale Veröffentlichungsnummer: WO 2016/097265

(56) Entgegenhaltungen:
- EP-A1- 1 769 889
- EP-A1- 1 769 889
- EP-A1- 2 502 636
- EP-A1- 2 502 636
- WO-A1-00/61199
- WO-A1-00/61199
- WO-A1-00/74735
- WO-A1-00/74735
- WO-A1-2010/040383
- WO-A1-2010/040383
- DE-A1- 4 409 659
- DE-A1- 4 409 659
- DE-U1- 9 421 818
- DE-U1- 9 421 818

## Beschreibung

Die Erfindung betrifft eine Vorrichtung zum schonenden Reinigen, Sterilisieren und Trocknen grosser Mengen von kleinen Teilen. Diese Teile können zum Beispiel Teile von Spritzen oder von Ampullen sein, also vorwiegend Teile, welche im medizinischen Bereich eingesetzt werden.

Für die Durchführung dieser Prozesse sind verschiedene Vorrichtungen und Verfahren bekannt, die aber mit Nachteilen behaftet sind, sei es im Bereich der Verfahren oder im Bereich der Ausführung der Vorrichtung.

Eine bekannte Vorrichtung besteht aus einem rotierenden fest installierten Behandlungsbehälter mit einer Siebplatte bzw. einem feinmaschigen Sieb, als Auflage für die kleinen Teile. Die Medien welche benötigt werden um die Behandlung vorzunehmen, durchströmen diese Siebplatte und erzeugen ein sog. Wirbelbett, welches die Behandlung bewirkt. Nach erfolgter Behandlung werden die Teile unter Einhaltung der Sterilität in sog. Transportbehälter transferiert und darin gelagert, bis diese an eine nachfolgende Vorrichtung übergeben werden.

Eine Anzahl weiterer bekannter Einrichtungen ist in den Dokumenten 2502636, EP1769889, WO 00/74735, WO00/61199, DE 4409659 oder DE9421818 beschrieben. Alle diese Dokumente beschreiben Vorrichtungen mit Behandlungsbehältern, die für den Transport auf Wagen platziert werden, aber für die Behandlung an eine Behandlungsmaschine angedockt werden müssen, wobei für den Behandlungsvorgang der Wagen entfernt wird, damit der Behälter im angedockten Zustand gedreht werden kann. Nach dem Behandlungsvorgang wird der Behälter wieder abgedockt und zurück in den Wagen gebracht.

Dieser Vorgang ist aufwändig, einerseits konstruktiv und mechanisch, andererseits sind solche An und Abdockvorgänge mit Risiko für den Bediener verbunden und unpraktisch. Ein weiterer Nachteil dieser Einrichtung ist, dass die Drehbarkeit des Behälters - welche für die Behandlung zwingend ist - nur an der Behandlungsmaschine möglich ist.
Zudem ist die Inspektion des Behälters an derartigen Einrichtungen aufwändig. Da der Behälter im Wagen nicht gedreht werden kann, muss der Behälter z.B. mit einer separaten Hubvorrichtung angehoben und gedreht werden, damit man in den Behälter sehen kann.

Auch das Beladen dieser bekannten Einrichtung gestaltet sich schwierig. Ausserdem ist wegen der begrenzten Drehbarkeit des Behälters die vollständige Entleerung auf der Überlaufseite nicht durchführbar. Die Waschflüssigkeit muss dann nach unten entleert werden und damit wird die Siebplatte und die saubere Einlaufseite mit z.B. Partikeln verschmutzt.

Ebenfalls ist es umständlich an den bekannten Einrichtungen einen Chargenverlust zu beheben. Da der Behälter im Wagen nicht gedreht werden kann, muss der Behälter z.B. mit einer separaten Hubvorrichtung angehoben und in die Entladeposition gedreht werden, damit die verlorene Charge entleert werden kann.

Der Erfindung liegt die Aufgabe zugrunde, die erwähnten Nachteile des Standes der Technik zu vermeiden.

Erfindungsgemäss wird dies erreicht durch eine Vorrichtung der eingangs genannten Art, die sich durch die kennzeichnenden Merkmale des Anspruchs 1 auszeichnet.

Im Folgenden wird anhand der beiliegenden Zeichnungen ein bevorzugtes Ausführungsbeispiel der Erfindung beschrieben. Es zeigen:

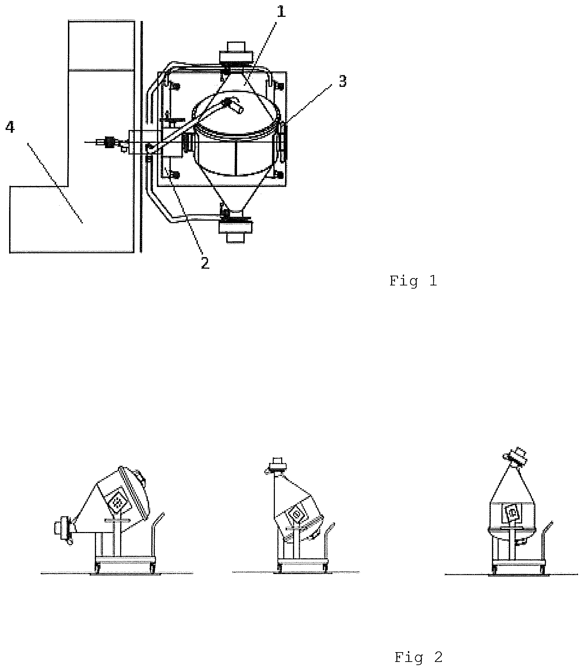

- Fig. 1: eine Draufsicht auf eine Vorrichtung nach der Erfindung
- Fig. 2: Seitenansichten derselben Vorrichtung mit verschiedenen Behälterpositionen.
- Fig. 3: die Vorrichtung mit dem Behälter in Entlee-rungsposition
- Fig. 4: die Vorrichtung mit dem Behälter in Inspektionsposition

Die in den Zeichnungen dargestellte Vorrichtung besteht aus einem Behandlungsbehälter 1 und einem Transportwagen 2, mit dem der Behandlungsbehälter fest verbunden ist, d.h. ein Behälter und ein Wagen bilden jeweils eine Einheit. Auf dem Wagen ist der Behälter mittels Lagervorrichtungen 3 drehbar gelagert. Die Lager sind so ausgebildet, dass die Behälterachse um 240° gedreht werden kann. Ausserdem ist eines der Lager mit an sich bekannten Mitteln zum Andocken des Behälters an eine Behandlungsstation 4 ausgestattet.

Durch sorgfältiges Drehen des Behälters auf dem Wagen wird eine schonende Behandlung der kleinen Teile erreicht werden. Die Entleerung von Restwasser und Kondensat, sowie auch die Entleerung von Hohlräumen der kleinen Teile, welche für eine gleichbleibende Behandlungsqualität notwendig ist, erfolgt ebenfalls durch sorgfältiges Drehen des Behälters. Für eine vollständige Entleerung kann die Behälterachse im Wagen auf minus 150° gedreht werden, und das Waschwasser kann mit Druckluft leergedrückt werden. Durch diese Entleermöglichkeit gelingt es die Partikel welche beim Waschen ausgeschwemmt wurden restlos zu entleeren und zwar ohne dass ein Rückfliessen des Waschmediums stattfindet.

Wenn das Wirbelbett abgestellt wird (Wirbelluft ab), befindet sich noch genügend Luftüberdruck unterhalb des Siebes, sodass das nachströmende Wasser die Luft verdrängt und keine Rückströmung in Richtung des Siebes stattfinden kann.

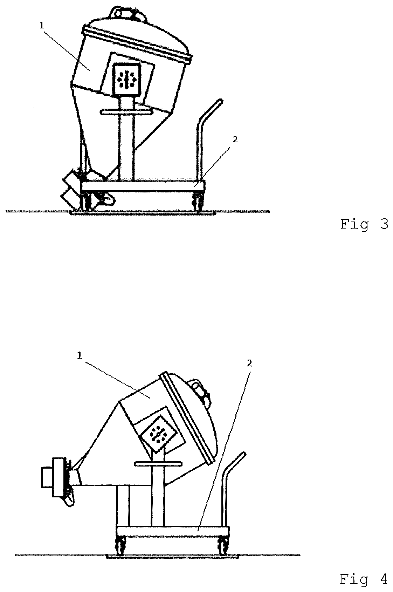

Unmittelbar nach dem Waschvorgang wird die Maschine in die in Fig. 3 gezeigte Entleerposition gedreht, so dass das Waschwasser vollständig auf die Abwasserseite herausgedrückt werden kann. Dadurch dass der Behälter vollständig nach unten entleerbar ist, wird die saubere Zuleitung und die Siebplatte nicht mit verschmutztem Wasser durchfahren.

Der Wagen dient zusätzlich als Inspektionsvorrichtung. Der Behälter kann im Wagen in die in Fig. 4 gezeigte Position nach unten gedreht werden, sodass eine gute Einsicht in den Behälter möglich ist, um eine Inspektion des inneren Behälters vorzunehmen. Falls sich noch verbleibende Teile im Behälter befinden, können diese durch weiteres Herunterdrehen des Behälters im Wagen entleert werden.

Bei einem Chargenverlust kann der Behälter auf dem Wagen in die Entleerstellung gedreht werden und die kleinen Teile können ohne zusätzliche Einrichtung nach unten entleert werden.

## Patentansprüche

1. Vorrichtung zum schonenden Reinigen, Sterilisieren und Trocknen grosser Mengen von kleinen Teilen mit einem Behälter (1), in welchem die Behandlung der Kleinteile durchgeführt wird, **dadurch gekennzeichnet, dass** der Behälter fest mit einem Wagen (2) verbunden ist, der Wagen mit Lagervorrichtungen (3) versehen ist, in denen der Behälter so gelagert ist, dass die Behälterachse drehbar ist, derart, dass der Behälter auf dem Wagen verbleibend an eine Behandlungsstation (4)andockbar ist.

## Claims

1. Device for gentle cleaning, sterilizing and drying of large quantities of small parts, the device having a container (1) in which the treatment of the small parts is carried out, **characterized in that** the container is permanently connected to a trolley (2), the trolley being equipped with mounting devices (3) in which the container is mounted such that the container axis is rotatable, in such a way that the container can be docked to a treatment station (4) while remaining on the trolley.

## Revendications

1. Dispositif de nettoyage, de stérilisation, et de séchage en douceur de grandes quantités de petites pièces à l'aide d'un récipient (1) dans lequel le traitement des petites pièces est effectué, **caractérisé en ce que** le récipient est solidement relié à un chariot (2), le chariot étant pourvu de dispositifs de stockage (3) dans lesquels le récipient est disposé de manière à ce que l'axe du récipient soit pivotant, de sorte que le récipient, reposant sur le chariot, peut être arrimé à une station de traitement (4).
